# FASCICULE DE BREVET EUROPEEN

(11) **EP 1 102 530 B1**
(45) Date de publication et mention de la délivrance du brevet: **02.03.2005**
(21) Numéro de dépôt: 99932972.5
(22) Date de dépôt: 26.07.1999
(51) Int. Cl.: A01K 67/027, C07K 14/47, A61K 49/00

(54) **UTILISATION D'UN MODELE ANIMAL DEFICIENT EN P53 ET PRESENTANT UNE DEFICIENCE DE LA MEMOIRE ET/OU DES TROUBLES COMPORTEMENTAUX A DES FINS THERAPEUTIQUES**
VERWENDUNG EINES TIERMODELLS, DAS P53-DEFIZIENT IST UND DESSEN ERINNERUNGSVERMÖGEN UND/ODER VERHALTEN GESTÖRT IST, ZUR ENTWICKLUNG VON THERAPEUTIKA
USE OF AN ANIMAL MODEL DEFICIENT IN P53 AND HAVING MEMORY DEFICIENCY AND/OR BEHAVIOURAL DISORDERS FOR THERAPEUTIC PURPOSES

(30) Priorité: 05.08.1998 FR 9810076
(43) Date de publication de la demande: 30.05.2001
(73) Titulaire: Molecular Engines Laboratories, 75011 Paris (FR)
(72) Inventeur: AMSON, Robert, F-75005 Paris (FR); LASSALLE, Jean-Michel, F-31450 Montbrun-Lauragais (FR); TELERMAN, Adam, F-75007 Paris (FR)
(74) Mandataire: Warcoin, Jacques
(86) Numéro de dépôt international: PCT/FR1999/001828
(87) Numéro de publication internationale: WO 2000/007438

(56) Documents cités:
- WO-A-92/11874
- WO-A-95/09916
- WO-A-95/19367
- SAH V ET AL: "A subset of p53-deficient embryos exhibit exencephaly" NATURE GENETICS, vol. 10, juin 1995 (1995-06), pages 175-180, XP002101318 cité dans la demande
- ROPERCH JP ET AL: "Inhibition of presenilin 1 expression is promoted by p53 and p21-WAF1 and results in apoptosis and tumor suppression" NATURE MEDICINE, vol. 4, no. 7, juillet 1998 (1998-07), pages 835-838, XP002122379 cité dans la demande
- JIANG Y H ET AL: "Mutation of the Angelman ubiquitin ligase in mice causes increased cytoplasmic p53 and deficits of contextual learning and long-term potentiation" NEURON, (1998 OCT) 21 (4) 799-811, XP002122380

## Description

La présente invention concerne un modèle animal souris présentant une déficience de la mémoire et/ou des troubles comportementaux liés à l'anxiété. Elle concerne également l'utilisation de ce modèle animal pour le criblage et la caractérisation de molécules susceptibles d'agir sur la mémoire et/ou l'anxiété.

Les inventeurs ont en effet établi une relation entre un défaut de fonctionnalité de la voie moléculaire du gène p53 d'une part et des troubles de la mémoire et/ou comportementaux liés à l'anxiété d'autre part.

Alors que le rôle du gène p53 en tant que suppresseur de tumeurs a largement été établi par une série d'études (Levine et al., 1991 ; Eliyahu et al., 1989 ; Michalovitz et al., 1990 ; Hollstein et al., 1991), les investigations sur les souris déficientes en p53 succombant rapidement à la néoplasie ont contribué à renforcer cette observation (Donchower et al., 1992). De façon intéressante, à un niveau particulier de leur développement, une partie significative de ces souris présente des altérations majeures incluant la fermeture anormale du tube neural (Sah et al., 1995 ; Armstrong et al., 1995) conduisant à une exencéphalie et ensuite une anencéphalie.

Les inventeurs se sont donc attachés à étudier des souris présentant une déficience au niveau de leur gène p53 mais nées avec un système nerveux central (CNS) intègre. Aucune étude précédente n'a décrit une quelconque anomalie histopathologique flagrante, cependant, ceci n'exclut pas le fait que le gène p53 non fonctionnel puisse influencer, à un niveau moléculaire, les voies de transduction donnant lieu à un fonctionnement anormal du système nerveux central.

Les inventeurs se sont donc attachés à déterminer si des souris homozygotes ou hétérozygotes quant à la déficience de leur gène p53 présentaient une quelconque particularité au niveau de leur comportement neurologique et cognitif.

Deux groupes d'animaux ont été étudiés, de jeunes souris homozygotes p53-/- (c'est-à-dire dont les deux allèles du gène p53 ne sont pas fonctionnels) et des souris adultes hétérozygotes p53-/+ (dont un seul des deux allèles du gène p53 n'est pas fonctionnel). Ce choix a été dicté par le fait que les souris p53-/- homozygotes (ou souris dites « knock out ») développent des tumeurs à un stade précoce de leur vie tandis que les souris hétérozygotes développent des tumeurs plus tardivement. Les expérimentations ont donc été réalisées pendant une période de temps où l'animal n'est pas malade puisque les souris portant des tumeurs peuvent avoir un comportement anormal ne résultant pas d'un dysfonctionnement neurologique mais plutôt du fait qu'elles souffrent de leurs tumeurs. Les souris déficientes au niveau de leur gène p53 et utilisées dans le cadre de présente invention sont commercialisées par TACONIC FARMS, USA.

Deux tests ci-dessous présentés en détail ont été réalisés, à savoir la piscine de Morris et l'Open-Field.

Le test de la piscine de Morris (Morris et al., 1981, Morris et al., 1984), dans lequel l'apprentissage spatial et la mémoire sont évalués, a été utilisé avec succès dans des études récentes notamment sur des souris modèles présentant les symptômes de la maladie d'Alzheimer (Nalbantoglu et al., 1997, Hsiao et al., 1996).

Dans le test de l'Open-Field, des paramètres comportementaux sont mesurés (Archer et al., 1973, Walsh et al., 1976). Cette procédure a déjà été utilisée dans le cadre de l'évaluation du profil psychologique de souris présentant un comportement associé à la peur (Gershenfeld et al., 1997) ainsi que pour étudier les effets sur le comportement du gène inactivé codant pour le récepteur de l'oestrogène (Ogawa et al., 1997).

Ces tests ont été réalisés en « aveugle » en ce qui concerne l'âge et le génotype des souris.

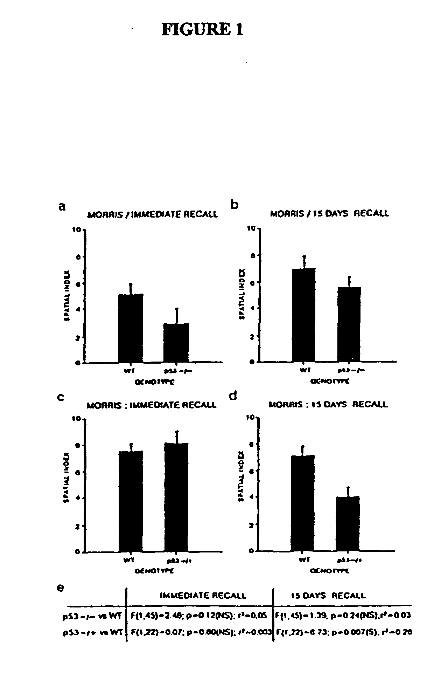

La **figure 1** rend compte des résultats obtenus par le test de la piscine de Morris. Il rend compte de l'indice de biais spatial en fonction du génotype des souris. Les figures la et 1b concernent les souris jeunes homozygotes p53-/- et les figures 1c et 1d concernent les souris hétérozygotes adultes p53 -/+. Dans les deux cas, l'index de biais spatial est calculé immédiatement après l'apprentissage de la composante procédurale du test puis quinze jours après sans entraînement supplémentaire.

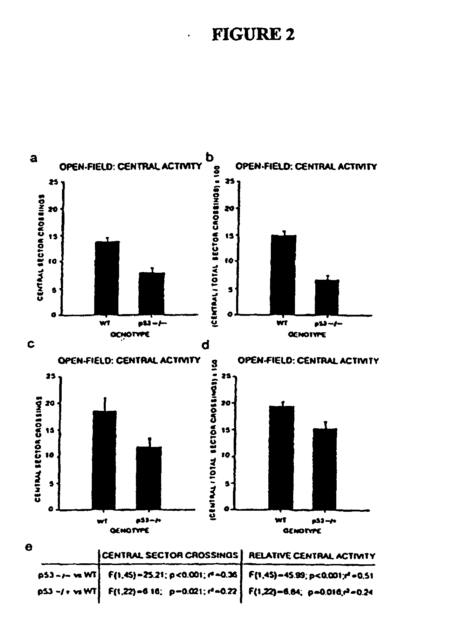

La **figure 2** rend compte des résultats obtenus par le test de l'Open-Field consistant à compter le nombre de fois où la souris traverse la partie centrale du champ sachant que l'anxiété qu'elle peut manifester est susceptible de l'en empêcher. Les figures 2a et 2c rendent compte du nombre de traversées du secteur central par les souris p53 -/- et p53 -/+ respectivement comparativement aux souris contrôles. Les figures 2b et 2d représentent le pourcentage du nombre de traversées du secteur central par rapport au nombre total de traversées des mêmes groupes de souris.

Les figures 1e et 2e concernent l'analyse de la variance (ANOVA) au moyen d'un schéma de mesures répétées, relativement au comportement des souris. La corrélation au carré (r²) a été calculée pour expliquer la part de la variance résultant de la déficience du gène p53.

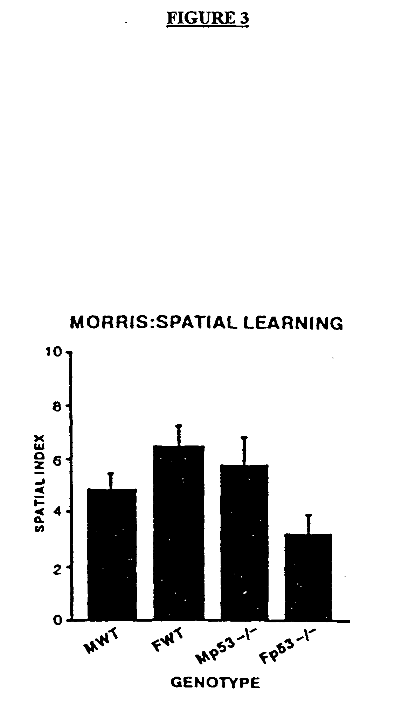

La **figure 3** rend également compte des résultats obtenus par le test de la piscine de Morris en fonction du sexe des souris. Une fois dans l'eau, les souris doivent apprendre à naviguer vers la plate-forme invisible. Après la fin de l'apprentissage, les souris sont soumises au test de l'indice de biais spatial (F = Femelle, M = Mâle, WT = sauvage). Les deux modes d'analyse de la variance ont révélé une interaction du sexe génotypique significative (F(1,43)=11,63 ; p=0,001 ; r²=0,26). Scheffé post-hoc test : Fp53 -/- versus FWT = 3,25 ; p = 0,01 ; Fp53 -/- versus Mp53 -/- = 2,77 ; p = 0,043.

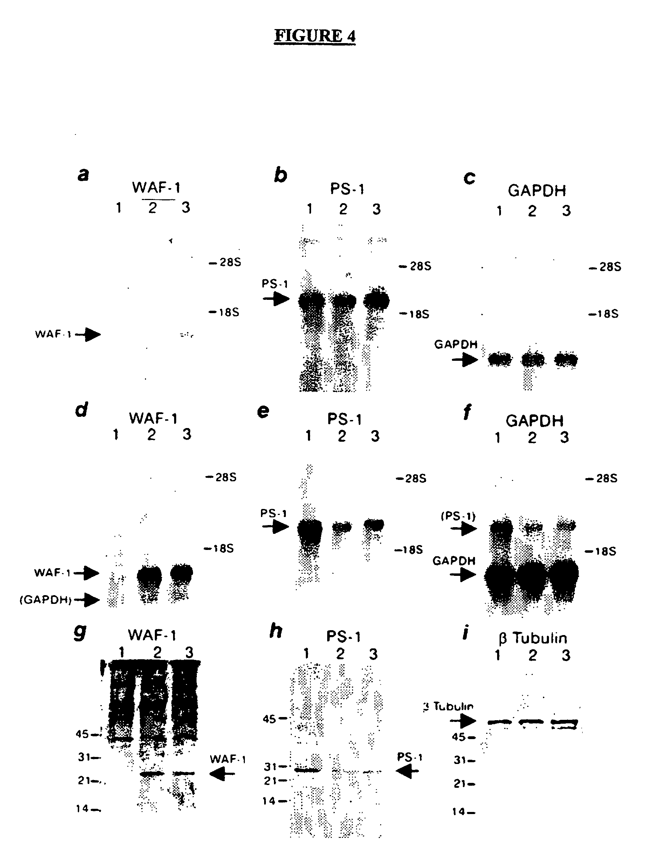

La **figure 4** illustre l'activation de p21Waf1 et la répression de PS1 dans le cerveau des souris adultes p53 -/- (il s'agit des souris p53 -/- ayant effectué jeunes les tests de la piscine de Morris et de l'Open-field tels que ci-dessus reportés. A l'analyse, il s'est avéré que ces souris n'avaient pas encore développé de tumeurs).

L'analyse par Northern blot (a-f) de Waf1, PS1 et GAPDH (en tant que contrôle) est réalisée sur des cerveaux de souris jeunes (a-c) et adultes (d-f). La bande 1 correspond à la souris contrôle, les bandes 2-3 aux souris p53 knock-out mâles et femelles. (a-c) correspondent au même Northem blot hybridé avec des sondes différentes. Aucune différence n'a été notée dans l'expression de Waf1 et de PS1 entre le contrôle et les souris knock-out. Pour les souris adultes, l'hybridation est également réalisée sur le même Northern blot (d-f). Les parenthèses pour (GAPDH) et (PS1) indiquent ce qui reste des sondes après la déshybridation dans des conditions non stringentes. Une induction forte de p21 Waf1 (d) et une répression de PS1 (e) sont notées chez les souris p53 déficientes.

L'analyse par Western blot est réalisée sur des extraits protéiques à partir des cerveaux des mêmes animaux adultes (g-i), avec des anticorps anti-Waf1, anti-PS1 et anti-bêta tubuline (en tant que contrôle). La bande 1 correspond à la souris contrôle ; les bandes 2-3 aux souris p53 knock-out. Une expression accrue de p21Waf1 et diminuée de PS1 sont observées chez les souris p53 déficientes.

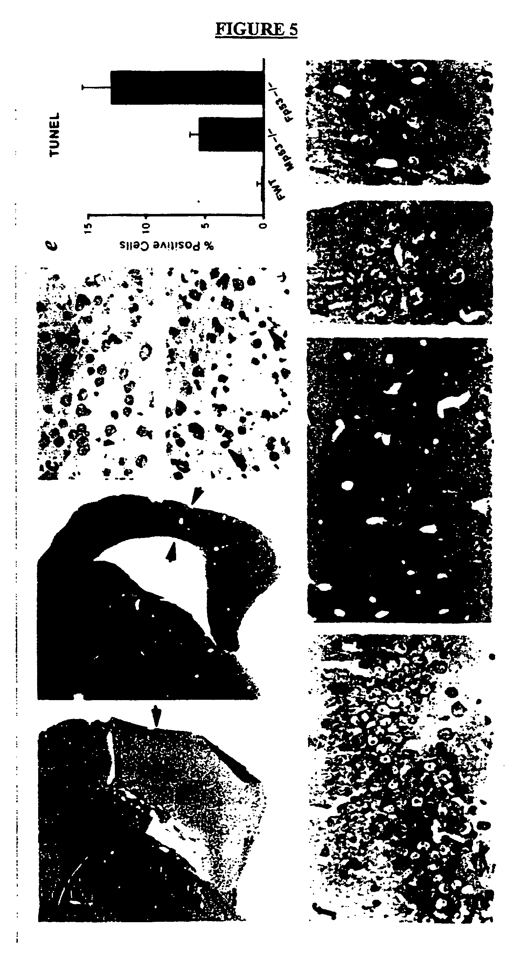

La **figure 5** illustre l'apoptose et l'accumulation intracytoplasmique d'amyloïde bêta 42 dans les cerveaux de souris adultes p53 -/- telles que définies ci-dessus.
(a) et (b) représentent un marquage de l'isocortex à l'haématoxyline et à l'éosine chez une souris contrôle et une souris knock-out respectivement. Les flèches indiquent un amincissement de l'isocortex chez la souris knock-out (b). Le grossissement est de 6,6 par rapport à l'origine.
(c) et (d) représentent le marquage par la technique TUNEL et le contre-marquage par l'haématoxyline de Harris chez le contrôle (c) et chez la souris knock-out (d). Chez (d), on observe deux noyaux positifs par la technique TUNEL (flèches). Le grossissement est de 132.
(e) illustre la quantification par la technique TUNEL.
(f-i) illustrent l'analyse immunohistochimique utilisant des anticorps spécifiques anti-amyloïde bêta 42. f et h correspondent aux souris contrôles, g et i sont des souris p53 knock-out qui montrent une accumulation intracytoplasmique d'amyloïde bêta 42. (f-g: grossissement x 40, h-1 grossissement x 100).

### Résultats du test de la piscine de Morris

Les jeunes souris p53-/- et les souris adultes p53-/+ satisfont au test d'une manière comparable aux souris contrôle à âge égal. Ceci indique que l'apprentissage spatial de ces souris n'est pas affecté par une quelconque déficience de leur gène p53 (figure 1a et 1c). De façon à mesurer la mémoire de ces souris, elles ont à nouveau été soumises au test de la piscine de Morris après une période de deux semaines et sans entraînement supplémentaire. Les jeunes souris p53-/- présentent un index spatial plus faible mais pas significativement différent de celui montré par les souris contrôle à âge égal (figure 1b). D'un autre côté, les souris adultes p53-/+ présentent un index spatial significativement inférieur à celui présenté par les souris contrôle (figure 1d).

Ces résultats montrent qu'une déficience de la mémoire est présente dans ce groupe d'animaux, puisque le véritable niveau de l'index spatial, après une période de quinze jours, dépend seulement de leur capacité à se remémorer le test. Un examen histologique du cerveau des souris adultes p53-/+ a été réalisé mais n'a pas révélé d'anomalie (donnée non montrée). Le fait que cette déficience de la mémoire soit seulement visible chez la souris adulte p53-/+ et pas chez la jeune souris p53-/- suggère qu'un facteur dépendant de l'âge, c'est-à-dire l'étape de maturation du cerveau, peut jouer un rôle dans la détection de la déficience de la mémoire. Quand bien même la plupart des études de ce type visant à détecter des anomalies comportementales ou au niveau de la mémoire, dans les maladies neurodégénératrices, sont réalisées sur des souris adultes, des souris homozygotes adultes ne pourraient pas être étudiées en raison du développement tumoral et c'est la raison pour laquelle l'étude a porté sur des souris hétérozygotes.

### Résultats du test de l'Open-Field

Les jeunes souris p53-/- (figure 2a) et adultes p53-/+ (figure 2c) montrent un nombre significativement inférieur de traversées du secteur central comparé au groupe de souris contrôle à âge égal. Puisque le nombre de traversées du secteur central est proportionnel à la capacité des souris à surmonter leur peur de s'éloigner de la paroi, pour explorer un territoire inconnu, ces résultats suggèrent que les souris déficientes en p53 sont plus thigmotaxiques que les souris correspondantes normales (c'est-à-dire qu'elles présentent une plus forte tendance à longer les parois). De façon à exclure la possibilité qu'un nombre faible de traversées du secteur central pouvant être dû à une activité locomotrice globalement diminuée, le nombre de traversées du secteur central a été exprimé comme un pourcentage du total des traversées. Cette activité centrale relative est également significativement diminuée dans les deux groupes de souris déficientes en p53 (figure 2b et 2d).

Les Inventeurs se sont ensuite attachés à approfondir les résultats des tests de la piscine de Morris tels que reportés ci-dessus obtenus pour les souris homozygotes p53 -/-, en fonction de leur sexe. Ils ont donc séparé les mesures d'index spatial obtenues pour les souris mâles de ceux obtenues pour les souris femelles et les ont comparées au groupe correspondant contrôle (Figure 3).

Tandis que les mâles p53 -/- ne présentent pas de différence significative avec les contrôles sauvages mâles et femelles, les femelles p53 -/montrent une performance significativement inférieure au contrôle, ceci indiquant des capacités à mémoriser et un apprentissage spatial moindres.

Les Inventeurs ont ensuite poursuivi leurs investigations afin de déterminer si les perturbations des fonctions du système nerveux central chez les souris p53 knock-out étaient corrélées avec des changements de l'expression dans le cerveau de p21Waf1 et PS1 puisque ces deux gènes sont régulés par p53 sauvage et qu'ils sont considérés comme modulateurs de l'apoptose. Ils ont mis en évidence des différences frappantes corrélées à l'âge. Les jeunes souris p53 -/- (âgés de 2 mois) présentent le même niveau d'ARNm correspondant à p21Waf1 et PS1 que les souris contrôles (Figure 4 a-c). Au contraire, les souris adultes p53 -/- (le même groupe sur lequel les tests de la piscine de Morris et de l'Open-field ont été réalisés deux mois plus tôt) présentent une forte augmentation de l'expression de p21Waf1 et de forte répression de l'expression de PS 1 (Figure 4 d-f). Compte tenu du fait que PS 1 subit un clivage protéolytique, les Inventeurs on testé son expression au niveau protéique. Comme montré sur la Figure 4h, l'expression de la protéine PS1 est très nettement diminuée chez les souris p53 knock-out. Les Inventeurs ont également testé les souris p53 knock-out concernant des mutations dans la région codante de l'ADNc de PS1. Rien n'a été trouvé. Ces observations suggèrent que les souris p53 knock-out plus âgées surcompensent la perte de p53 par une surexpression de p21Waf avec en conséquence une répression de PS1. Les études antérieures des Inventeurs ont montré que l'expression accrue de p21Waf1 résultait de la répression de PS1 avec une induction de l'apoptose et que l'inhibition de la production de PS1 par un ADNc antisens induisait l'apoptose. Ceci corrobore la notion selon laquelle la diminution de l'expression de PS1 dans le cerveau pourrait causer des altérations dans le fonctionnement du système nerveux central par l'intermédiaire d'une augmentation de l'apoptose.

Le fait que de jeunes souris p53 knock-out présentent déjà des déficiences concernant l'apprentissage, la mémoire et le comportement avant une diminution mesurable de l'expression de PS 1 est similaire à ce qui est trouvé dans les maladies neurodégénératives. Des anormalités dans le comportement peuvent s'observer chez des patients atteints de la maladie d' Alzheimer longtemps avant le stade terminal de la maladie dans laquelle il y a neurodégénérescence. Ces données suggèrent un procédé progressant lentement dans lequel les anormalités de la mémoire et du comportement ont lieu avant qu'une répression de PS1 ne devienne mesurable.

Les analyses histopathologiques combinées avec la technique TUNEL pour évaluer l'apoptose ont été réalisées sur le même groupe d'animaux âgés montrant une diminution de l'expression de PS1 comme ci-dessus indiqué. Trois des quatre souris n'ont montré aucune anormalité pathologique frappante. Une souris knock-out (femelle) présentait quant à elle un amincissement de plus de 50 % de l'isocortex avec un ventricule très élargi comparé aux animaux contrôles (Figure 5a-b). L'expérimentation par TUNEL a montré des lésions apoptotiques discrètes (Figure 5 d) dans le cerveau sans apoptose massive. La quantification des cellules positives par TUNEL (Figure 5e) a indiqué que, tandis que dans les cerveaux contrôles seulement un cas montrait cinq cellules positives sur 1 740 comptées, les souris p53 -/- avaient jusqu'à 5,5 % (25/465) et 13% (39/305) de cellules positives par TUNEL chez les animaux mâles et femelles respectivement (Figure 5e).

Puisque les patients avec la maladie d'Alzheimer et les souris transgéniques avec des mutations dans PS1 montrent une neurodégénérescence avec apoptose et accumulation d'amyloïde bêta 42, les Inventeurs ont ensuite recherché la présence d'amyloïde bêta 42 dans le cerveau des souris p53 knock-out. Comme montré dans les Figures 5 h-i, les souris déficientes en p53 montrent des niveaux élevés d'amyloïde bêta 42 dans le compartiment cytoplasmique.

En conclusion, ces expérimentations indiquent que l'expression intacte de p53 est nécessaire pour assurer les fonctions complexes du système nerveux central. Ces fonctions incluent la mémoire et un comportement standard dans des situations anxiogènes. Le mécanisme précis selon lequel la perte de la fonctionnalité de p53 interfère avec la mémoire et le comportement peut être dû à une dérégulation de la voie métabolique p53 au niveau moléculaire.

De plus, il a été montré que les souris p53 -/- qui présentaient des déficiences au niveau de la mémoire, dans leur habilité à apprendre, ainsi qu'un comportement anormal avaient des lésions apoptotiques discrètes dans leur cerveau. Ces altérations semblent impliquer une sur-compensation de la déficience de p53 à long terme et dépendant de l'âge par une augmentation de l'expression de p21Waf1 qui conduit à une répression de PS1. Ceci, en retour, compromet l'effet anti-apoptotique de PS1. Ces données indiquent que la perte de la fonction de PS1 par l'intermédiaire, soit de mutations telles que montrées dans les maladies d'Alzheimer familiales précoces, soit par la dérégulation de l'expression comme c'est le cas chez les souris p53 déficientes, active le programme de mort cellulaire. Ceci est couplé avec le métabolisme altéré de l'APP (Amyloïd Precursor Protein) conduisant à l'accumulation de l'amyloïde bêta 42. Ainsi, les Inventeurs ont mis à jour la base à de nouvelles stratégies pour le traitement de maladies neurodégénératives.

Ainsi, de façon surprenante, les inventeurs ont mis en évidence qu'une souris déficiente au niveau de son gène p53 présentait des troubles de l'apprentissage, de la mémoire et/ou des troubles comportementaux tels que l'anxiété.

Par conséquent, la présente invention concerne un modèle animal souris, présentant une déficience de la mémoire, l'animal en question ayant l'un au moins des allèles de son gène p53 non fonctionnel. C'est le cas des souris p53-/+.

Le modèle en question peut en outre présenter des troubles du comportement tels que l'anxiété, et dans ce cas, ce sont les deux allèles du gène p53 de l'animal qui ne sont pas fonctionnels. C'est le cas des souris homozygotes p53-/-, mais également comme indiqué plus haut, des souris p53 -/+ qui présentent également des troubles comportementaux dans une situation anxiogène.

Ainsi, le modèle animal souris en question peut être utilisé pour le criblage de molécules susceptibles de présenter une activité anxiolytique et/ou de restaurer au moins en partie la mémoire.

En effet, après avoir fait subir un quelconque test d'apprentissage à des souris p53-/+, on sait que deux semaines après, elles présentent une déficience de la mémoire. On les traite alors avec une molécule (de préférence soupçonnée d'agir sur la mémoire), et on soumet à nouveau les souris traitées audit test. La molécule ne sera retenue pour des investigations supplémentaires que si elle a permis de restaurer significativement la mémoire desdites souris.

Dans le cadre de tests classiquement effectués dans ce domaine, le modèle animal souris conforme à l'invention peut également être utilisé pour déterminer les caractéristiques d'une molécule dont on sait déjà qu'elle est susceptible d'agir sur l'anxiété et/ou la mémoire, les caractéristiques concernant la pharmacodynamie, la pharmacocinétique, la toxicité de ces molécules...

L'invention ne se limite pas à la description ci-dessus et les résultats obtenus dans le cadre des susdites expérimentations seront mieux compris au moyen de la présentation ci-dessous des deux tests auxquels ont été soumises les souris.

### METHODES : Mesures comportementales

**La piscine de Morris** (Morris, 1981). La piscine circulaire en PVC couleur ivoire de 70 cm de diamètre et 30 cm de hauteur, est remplie d'une eau à 22 ± 1°C, colorée en blanc par adjonction de colorant Opacifier 631®, jusqu'à une hauteur de 12 cm en dessous du bord de la paroi. Une plate-forme circulaire de 5 cm de diamètre servant de but est immergée sous 0,5 cm d'eau, à 7 cm de la paroi. Le dispositif est placé dans une pièce rectangulaire. Deux repères rectangulaires (50 x 30 cm), un noir et un rayé noir et blanc, sont accrochés sur deux murs adjacents, à 1,5 mètre de la piscine. Une caméra vidéo, placée à la verticale de la piscine permet d'enregistrer les trajets des souris sur un magnétoscope en vue de les analyser ultérieurement. L'expérimentateur est dissimulé derrière un rideau blanc. Placées dans l'eau, face à la paroi, dans un quadrant qui varie de manière pseudo-aléatoire d'un essai à l'autre, les souris doivent apprendre à naviguer vers la plate-forme en utilisant les indices visuels distaux disponibles dans la pièce. Après une séance d'entraînement de trois essais destinée à l'apprentissage de la composante procédurale du test, les souris sont soumises à trois essais consécutifs par jour pendant 4 jours. Le temps maximum autorisé pour atteindre la plate-forme a été fixé à 60 secondes. Si une souris ne peut atteindre la plate-forme dans ce laps de temps, elle est guidée jusqu'à elle par l'expérimentateur. La somme des trois temps de latence constitue le score de la séance. Après le dernier essai de la troisième séance, la souris est soumise a un test de biais spatial destiné à vérifier la précision de l'apprentissage spatial (mémoire à court terme). La plate-forme est alors retirée du dispositif et la souris, partant du quadrant opposé, doit nager à sa recherche pendant une minute. Le trajet est enregistré au magnétoscope et un indice de biais spatial est alors calculé. Il correspond à la différence du nombre de franchissement d'un anneau de 8 cm entourant la position de la plate-forme et du nombre moyen de franchissements des trois annuli placés symétriquement dans les trois autres quadrants. Un autre test de biais spatial est effectué après un délai de 15 jours sans entraînement supplémentaire (mémoire à long terme).

**Open-field** (Archer, 1973 ; Walsh et Cummins, 1976). Les souris sont introduites individuellement dans un cylindre en PVC gris de 40 cm de diamètre et 30 cm de hauteur, placé sur une feuille de papier blanc au travers de laquelle on peut distinguer les traits qui divisent le plancher de l'open-field en 7 secteurs de même surface (1 secteur central et 6 secteurs périphériques). Ce dispositif, éclairé par une lumière blanche diffusant à travers un verre dépoli (125 lux) constitue pour la souris une situation modérément anxiogène. Les souris sont soumises à trois séances quotidiennes de 15 minutes, espacées de 45 minutes et ce, pendant 4 jours consécutifs. Les mesures comportementales sont effectuées pendant les 5 premières minutes de chaque séance. Elles consistent essentiellement à compter le nombre de fois où la souris « s'enhardit » à s'éloigner des parois latérales du cylindre pour traverser le secteur central de l'Open-Field.

### REFERENCES

Amson, R., et al. *Proc. Natl*. *Acad Sci. USA* **93**, 3953-3957 (1996)
Archer, J. *Animal Behaviour* **21**, 205-235 (1973)
Armstrong, J.F., Kaufman, M.H., Harrison, D.J. and Clarke, A.R. *Curr. Biol., 5,* 931-936 (1995)
Chapillon, P. and Roullet, P. *Developmental Psychobiology* **29**, 529-545 (1996) Donchower, L.A. et al. *Nature* **356**, 215-221 (1992)
Eliyahu, D., et al. *Proc. Natl. Acad Sci. USA* **86**, 8763- (1989)
Gershenfeld, H.K. and Paul, S.M. *Genomics* **46**, 1-8 (1997)
Hollstein, M., Sidransky, D., Vogelstein, B., Harris, C ; C. *Science* **253**, 49-53 (1991)
Hsiao, K. et al *Science* **274**, 99-102 (1996)
Levine, A.J., Momand, J. and Finlay, C.A., *Nature* **351**, 453- (1991)
Michalovitz, D., Halevy, O. and Oren, *M. Cell* **62**, 671- (1990)
Morris, R.G. M. *Learning & Motivation* **12**, 239-260 ( 1981)
Morris, R.G. M. J. Neurosci. *Meth*. **11**, 47-60 (1984)
Nalbantoglu, J. et al. *Nature* **387**, 500-505 (1997)
Nemani, M., et al. *Proc. Natl. Acad. Sci. USA* **93**, 9039-9042 (1996)
Ogawa, S., Lubahn, D.B., Korach, K.S. and Pfaff, D.W. *Proc. Natl. Acad Sci. USA* **94**, 1476-1481 (1997)
Roperch J.P., Alvaro V., Prieur S., Tuynder M., Nemani M., Lethrosne F., Piouffre L., Gendron M.G., Israeli D., Dausset J., Oren M., Amson R., and Telerman A. Inhibition of Presenilin 1 expression is promoted by p53 and p21WAF-1 and results in apoptosis and tumor suppression. *Nature Medicine* 1998 ; 4 : 835-838
Sah, V.P. et al. *Nat. Genet.* **10**, 175-180 (1995)
Walsh, R.N. and Cummins, R.A. *Psychological Bulletin* **83**, 482-504 (1976)

## Revendications

1. Utilisation d'un modèle animal souris présentant une déficience de la mémoire et/ou des troubles du comportement tels que l'anxiété, dont au moins l'un des allèles du gène p53 n'est pas fonctionnel, pour le criblage de molécules susceptibles de présenter une activité anxiolytique.

2. Utilisation d'un modèle animal tel que défini dans la revendication 1, pour déterminer les caractéristiques d'une molécule présentant une activité anxiolytique telle que la pharmacodynamie, la pharmacocinétique et la toxicité.

3. Utilisation d'un modèle animal selon la revendication 1 ou 2, **caractérisée en ce que** les deux allèles du gène p53 ne sont pas fonctionnels.

4. Utilisation d'un modèle animal tel que défini dans la revendication 1, pour le criblage de molécules susceptibles de restaurer au moins en partie la mémoire.

5. Utilisation d'un modèle animal tel que défini dans la revendication 1, pour déterminer les caractéristiques d'une molécule capable de restaurer au moins en partie la mémoire telles que la pharmacodynamie, la pharmacocinétique et la toxicité.

## Patentansprüche

1. Verwendung eines Maus-Tiermodells, welches eine Gedächtnisstörung und/oder Verhaltensstörungen, wie Angst, aufweist, bei welchem wenigstens eines der Allele des Gens p53 nicht funktionsfähig ist, für das Screenen von Molekülen, welche in der Lage sind, eine anxiolytische Aktivität aufzuweisen.

2. Verwendung eines Tiermodells, wie in Anspruch 1 definiert, um die Eigenschaften eines Moleküls, welches eine anxiolytische Aktivität aufweist, wie die Pharmakodynamik, die Pharmakokinetik und die Toxizität, zu bestimmen.

3. Verwendung eines Tiermodells nach Anspruch 1 oder 2, **dadurch gekennzeichnet, dass** die beiden Allele des Gens p53 nicht funktionsfähig sind.

4. Verwendung eines Tiermodells, wie in Anspruch 1 definiert, für das Screenen von Molekülen, welche in der Lage sind, das Gedächtnis wenigstens teilweise wiederherzustellen.

5. Verwendung eines Tiermodells, wie in Anspruch 1 definiert, um die Eigenschaften eines Moleküls, welches in der Lage ist, das Gedächtnis wenigstens teilweise wiederherzustellen, wie die Pharmakodynamik, die Pharmakokinetik und die Toxizität, zu bestimmen.

## Claims

1. Use of a mouse animal model showing memory deficiency and/or behavioural disorders such as anxiety, in which at least one of the alleles of the p53 gene is not functional, for the screening of molecules capable of exhibiting anxiolytic activity.

2. Use of an animal model as defined in Claim 1, for determining the characteristics of a molecule having an anxiolytic activity such as the pharmacodynamics, the pharmacokinetics and the toxicity.

3. Use of an animal modelr according to Claim 1 or 2, **characterized in that** the two alleles of the p53 gene are not functional.

4. Use of an animal model as defined in Claim 1, for the screening of molecules capable of at least partially restoring memory.

5. Use of an animal model as defined in Claim 1, for determining the characteristics of a molecule capable of at least partially restoring memory such as the pharmacodynamics, the pharmacokinetics and the toxicity.
